# EUROPEAN PATENT APPLICATION

(11) **EP 4 227 956 A1**
(43) Date of publication of application: **16.08.2023**
(21) Application number: 21877760.5
(22) Date of filing: 08.10.2021
(51) Int. Cl.: G16H 50/00

(54) **DISEASE DIAGNOSIS RESULT DETERMINATION DEVICE, DISEASE DIAGNOSIS RESULT DETERMINATION METHOD, AND PROGRAM**

(30) Priority: 09.10.2020 JP 2020171329
(71) Applicant: The University of Tokyo, Bunkyo-ku, Tokyo 113-8654 (JP)
(72) Inventor: SAKATANI Kaoru, Tokyo 113-8654 (JP)
(74) Representative: Peters, Sebastian Martinus
(86) International application number: PCT/JP2021/037418
(87) International publication number: WO 2022/075461

(57) **Abstract**

This disease diagnosis result determination device is provided with: a brain examination result determination unit that determines a result of brain examination concerning a second subject on the basis of medical checkup data concerning the second subject and a first learning result obtained by performing learning in advance of a relationship between medical checkup data concerning a first subject and a result of brain examination concerning the first subject;
and a disease diagnosis result determination unit that determines a disease diagnosis result concerning the second subject.

## Description

### TECHINCAL FIELD

The present invention relates to a disease diagnosis result determination device, a disease diagnosis result determination method, and a program.

This application claims priority to Japanese Patent Application 2020-171329 filed in Japan on October 9, 2020, the entire contents of which are incorporated by reference herein.

### BACKGROUND ART

As the population ages, the number of dementia patients is rapidly increasing and becoming a social problem. The early detection of cognitive impairment is important for the treatment and prevention of dementia. However, many cases are based on diagnosis of dementia having progressed. One of the reasons is that a large-scale screening examination method for cognitive impairment has not been established. Currently, questionnaire tests such as MMSE are used for screening examinations. This is because questionnaire tests, which are subjective examination methods, require to be conducted on a one-to-one basis. Therefore, such questionnaire tests cannot be used for large-scale screening in a short period of time. Imaging examinations such as MRI are highly capable of diagnosing dementia, but are not suitable for screening because they are expensive examination methods.

Hitherto, as methods for determining the risk of cognitive impairment, methods have been proposed that use machine learning to determine the risk of cognitive impairment based on basic blood examination data and age (PTL 1 and PTL 2). In the methods of determining the risk of cognitive impairment, described in PTL 1 and PTL 2, the relationship between a set of ages and the results of basic blood examinations and Mini-Mental State Examination (MMSE) scores, an MMSE being a questionnaire test for cognitive functions, is trained by machine learning, and an MMSE score is determined based on age and the results of a basic blood examination. In PTL 2, deep learning is used as the machine learning, and a set of ages and the results of basic blood examinations is input to an input layer, and an MMSE score is output from an output layer.

### CITATION LIST

### PATENT LITERATURE

[PTL 1] Japanese Patent Application Publication No. 2018-55333
[PTL 2] Japanese Patent Application Publication No. 2018-149168

### SUMMARY OF THE INVENTION

### TECHNICAL PROBLEM

The methods of determining the risk of cognitive impairment, described in PTL 1 and PTL 2, have the following problems. In the case where deep learning is used as the machine learning, the type of data (age, blood examination data) input to the input layer of a neural network used for the deep learning and the type of data output from the output layer (assessment of cognitive function through a questionnaire test such as MMSE) are limited, and the prediction accuracy of cognitive impairment is poor. Questionnaire tests such as MMSE are subjective assessment methods and may be inaccurate in assessment of cognitive function. In conventional deep learning, the process of predicting the data output from the output layer based on the data input to the input layer is a black box, which has been an obstacle for the application of deep learning to medical care.

Accordingly, there is a need to improve the accuracy of determination of the result of disease diagnosis.

The present invention has been made in view of the foregoing, and provides a disease diagnosis determination device, a disease diagnosis determination method, and a program, capable of improving the accuracy of determination of the result of disease diagnosis.

### SOLUTION TO THE PROBLEM

The present invention has been made in order to solve the above problems. A disease diagnosis result determination device according to one aspect of the present invention includes: a brain examination result determination unit configured to determine, based on a first learning result for which a relationship between health examination data of a first subject and a result of brain examination of the first subject is learned in advance and based on health examination data of a second subject, a result of brain examination of the second subject; and a disease diagnosis result determination unit configured to determine, based on a second learning result for which a relationship between a result of brain examination of a third subject and a result of disease diagnosis of the third subject is learned in advance and based on the result of brain examination of the second subject determined by the brain examination result determination unit, a result of disease diagnosis of the second subject.

Further, according to one aspect of the present invention, in the above-described disease diagnosis result determination device, the result of brain examination includes results of a plurality of types of brain examinations, the first learning result includes a plurality of types of learning results for the respective types of brain examinations, and the brain examination result determination unit is configured to determine, based on the plurality of types of learning results for the respective types of brain examinations and based on the health examination data of the second subject, each of the results of the plurality of types of brain examinations of the second subject.

Further, according to one aspect of the present invention, the above-described disease diagnosis result determination device further includes a brain examination result acquisition unit configured to acquire a result of brain examination obtained by performing one or some of the types of brain examinations of the second subject in advance, wherein the disease diagnosis result determination unit is configured to use the result of brain examination acquired by the brain examination result acquisition unit and make the determination for the one or some of the types of brain examinations, instead of the result of brain examination of the second subject determined by the brain examination result determination unit.

Further, according to one aspect of the present invention, in the above-described disease diagnosis result determination device, the brain examination result determination unit is configured to determine a result of brain examination of the second subject based on the first learning result and the health examination data from the second subject by using statistics of health examination data from a plurality of fourth subjects as part of the health examination data from the second subject.

According to one aspect of the present invention, the above-described disease diagnosis result determination device further includes a brain examination result output unit configured to output, after the disease diagnosis result determination unit determines the result of disease diagnosis, a set of the results of brain examination of the second subject used to determine the result of disease diagnosis and the result of disease diagnosis.

Further, a disease diagnosis result determination method according to one aspect of the present invention includes: a brain examination result determination step of determining, based on a first learning result for which a relationship between health examination data from a first subject and a result of brain examination of the first subject is learned in advance and based on health examination data from a second subject, a result of brain examination of the second subject; and a disease diagnosis result determination step of determining, based on a second learning result for which a relationship between a result of brain examination of a third subject and a result of disease diagnosis of the third subject is learned in advance and based on the result of brain examination of the second subject determined by the brain examination result determination step, a result of disease diagnosis of the second subject.

Further, a program according to one aspect of the present invention causes a computer to execute: a brain examination result determination step of determining, based on a first learning result for which a relationship between health examination data from a first subject and a result of brain examination of the first subject is learned in advance and based on health examination data from a second subject, a result of brain examination of the second subject; and a disease diagnosis result determination step of determining, based on a second learning result for which a relationship between a result of brain examination of a third subject and a result of disease diagnosis of the third subject is learned in advance and based on the result of brain examination of the second subject determined by the brain examination result determination step, a result of disease diagnosis of the second subject.

### ADVANTAGEOUS EFFECT OF THE INVENTION

According to the present invention, the accuracy of determination of the result of disease diagnosis can be improved.

### BRIEF DESCRIPTION OF THE DRAWINGS

Fig. 1 is a diagram illustrating an example of the configuration of a disease diagnosis result determination device according to an embodiment of the present invention.
Fig. 2 is a diagram illustrating an example of the configuration of a neural network used for machine learning according to the embodiment of the present invention.
Fig. 3 illustrates an example of a determination step of the disease diagnosis result determination device according to the embodiment of the present invention.
Fig. 4 is a diagram illustrating an example of the configuration of a disease diagnosis result determination device according to Modified Example 1 of the embodiment of the present invention.
Fig. 5 illustrates an example of a determination step of the disease diagnosis result determination device according to Modified Example 1 of the embodiment of the present invention.
Fig. 6 is a diagram illustrating an example of the configuration of a disease diagnosis result determination device according to Modified Example 2 of the embodiment of the present invention.
Fig. 7 illustrates an example of a determination step of the disease diagnosis result determination device according to Modified Example 2 of the embodiment of the present invention.
Fig. 8 illustrates an example of a next-generation preventive-medicine model according to an embodiment of the present invention.
Fig. 9 illustrates an example of a health future prediction chart according to an embodiment of the present invention.
Fig. 10 illustrates an example of a correlation between actual measured values (correct labels) and predicted values of GM-BHQ according to an example of the present invention.
Fig. 11 illustrates an example of the degrees of importance to prediction results for items included in blood examination data according to an example of the present invention.

### DESCRIPTION OF EMBODIMENTS

### Embodiment

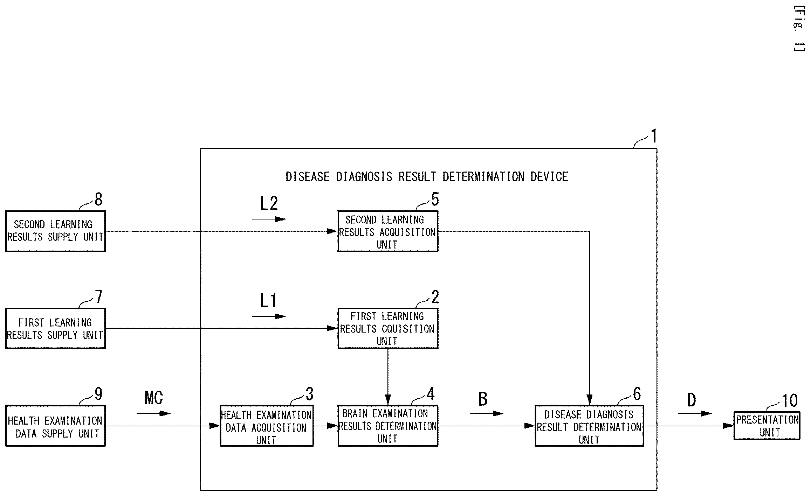

An embodiment of the present invention will be described below in detail with reference to the drawings. Fig. 1 is a diagram illustrating an example of the configuration of a disease diagnosis result determination device 1 according to the present embodiment. In response to receiving health examination data, the disease diagnosis result determination device 1 determines a result of cognitive impairment diagnosis based on first machine learning and second machine learning. In the present embodiment, each of the first machine learning and the second machine learning is deep learning by way of example. The disease diagnosis result determination device 1 uses so-called two-stage deep learning similar to a medical diagnosis process. In the stage of the first machine learning, the results of a brain examination method (a cognitive test such as mini mental state examination (MMSE), magnetic resonance imaging (MRI) diagnostic imaging, etc.) are predicted from health examination data based on the relationship between systemic disorders and brain disorders. Next, in the stage of the second machine learning, the risk of cognitive impairment is determined so that a doctor can make an integrated dementia diagnosis from the results of the brain examination method.

The disease diagnosis result determination device 1 includes a first learning results acquisition unit 2, a health examination data acquisition unit 3, a brain examination results determination unit 4, a second learning results acquisition unit 5, and a disease diagnosis result determination unit 6. The disease diagnosis result determination device 1 is, for example, a personal computer (PC). Note that the disease diagnosis result determination devices 1, 1a, and 1b may each be a portable terminal device such as a smartphone.

The first learning results acquisition unit 2 acquires first learning results L1 supplied from a first learning results supply unit 7. The first learning results L1 are results of the first machine learning that is performed in advance on the relationship between health examination data from a first subject P1 and results of brain examination of the first subject P1. The first machine learning is, for example, deep learning. The first learning results L1 include parameters (weights and biases) of a neural network used for the deep learning.

In the present embodiment, the results of brain examination include results of a plurality of types of brain examinations. The first learning results L1 include one or more types of learning results for the respective types of brain examinations. The plurality of types of learning results are referred to as brain examination learning results LB-i (i = 1, 2, ..., n: n is the number of types of brain examination). The results of brain examination include, for example, one or more of: the results of a questionnaire test on cognitive function such as MMSE; an index (VSRAD index, brain healthcare quotient (BHQ), etc.) from diagnostic imaging (MRI, CT, PET, etc.); lesion findings (cerebral infarction, cerebral atrophy, etc.); a dementia biomarker (amyloid β, etc.); and the like.

In the present embodiment, the results of a brain examination include, for example, three indices: an MMSE score; gray matter brain healthcare quotient (GM-BHQ), which is an index indicating the volume of brain gray matter; and fractional anisotropy brain healthcare quotient (FA-BHQ), which is an index expressing the integrity of nerve fiber of the cerebral white matter as fractional anisotropy. A brain examination learning result LB-1 is a learning result corresponding to an MMSE score. A brain examination learning result LB-2 is a learning result corresponding to a GM-BHQ. A brain examination learning result LB-3 is a learning result corresponding to an FA-BHQ.

Health examination data of the first subject P1 is also referred to as teacher health examination data. Further, the results of brain examination of the first subject P1 is also referred to as the teacher brain examination results. First subjects P1 are subjects for which the teacher health examination data and the teacher brain examination results are collected. The number of first subjects P1 is preferably a predetermined number to improve the accuracy of determination using machine learning, but may be one or more.

The health examination data may be of any examination item as long as it is from various types of health examinations (physical checkups), such as specific health examinations. Further, the health examination data may be data that can be obtained from the subject individually, not at various medical institutions, such as data measured by a healthcare monitoring device or a body composition monitor. The health examination data includes, for example, one or more of: age, results of basic blood examination, measurement results of physical functions (one or more of height, weight, waist circumference, BMI, blood pressure, pulse rate, etc.), medical history (one or more of medicine dosage history, smoking habits, etc.), family history, information indicating subjective symptoms (one or more of physical findings, neurological findings, etc.), examination results of physiological functions (one or more of an electrocardiogram, an electroencephalogram, hearing examination, balance examination, visual examination, taste examination, etc.), examination results of psychological functions (one or more of intelligence quotient, state trait anxiety, etc.), other biological information (one or more of fundus examination, digestive system examination, dental examination, spoken language ability, behavior analysis, etc.), and information indicating oriental medical findings (one or more of tongue examination, pulse examination, palpation, etc.).

The health examination data acquisition unit 3 acquires health examination data MC supplied from a health examination data supply unit 9. The health examination data MC is health examination data from the second subject P2. The second subject P2 is a subject for which a result of disease diagnosis is to be determined by the disease diagnosis result determination device 1. The data items included in the health examination data from the second subject P2 match the data items included in the health examination data from the first subject P1 described above. For example, if the health examination data from the first subject P1 is a set of age, results of basic blood examination, and results of electroencephalography of the first subject P1, the health examination data from the second subject P2 is a set of age, results of basic blood examination, and results of electroencephalography of the second subject P2.

The brain examination result determination unit 4 determines a brain examination result determination result B based on the first learning result L1 and the health examination data MC. The brain examination result determination result B is a result of brain examination of the second subject P2.

As described above, the first learning result L1 is a learning result based on the first machine learning, and thus the brain examination result determination unit 4 makes a determination based on the first machine learning.

Specifically, based on the brain examination learning result LB-i, which includes a plurality of types of learning results from the respective types of brain examinations, and based on the health examination data MC, the brain examination result determination unit 4 determines each of the results of the plurality of types of brain examinations of the second subject P2. The brain examination result determination result B includes a plurality of brain examination result determination results B-i (i = 1, 2, ..., n: n is the number of types of brain examinations) for the respective types of brain examinations. In the present embodiment, the brain examination result determination result B includes the brain examination result determination result B-1, which is a determination result of MMSE score, the brain examination result determination result B-2, which is a determination result of GM-BHQ, and the brain examination result determination result B-3, which is a determination result of FA-BHQ.

The second learning result acquisition unit 5 acquires a second learning result L2 supplied from a second learning result supply unit 8. The second learning results L2 are results of the second machine learning that is performed in advance on the relationship between results of brain examination of a third subject P3 and results of disease diagnosis of the third subject P3. The second machine learning is, for example, deep learning. The second learning results L2 include parameters (weights and biases) of a neural network used for the deep learning.

The results of brain examination of the third subject P3 is also referred to as the teacher brain examination results. Further, the results of disease diagnosis of the third subject P3 are also referred to as the teacher disease diagnosis results. Third subjects P3 are subjects for which the teacher brain examination results and the teacher disease diagnosis results are collected. The number of third subjects P3 is preferably a predetermined number to improve the accuracy of determination using machine learning, but may be one or more. The teacher disease diagnosis result is a result of disease diagnosis (cognitive impairment) in which a specialist makes a diagnosis based on the result of brain examination of the third subject P3. The result of disease diagnosis is classified into, for example, one of the classes of "normal", "mild cognitive impairment (MCI)", and "dementia".

The disease diagnosis result determination unit 6 determines a disease diagnosis result D based on the second learning result L2 and the brain examination result determination result B determined by the brain examination result determination unit 4. The disease diagnosis result D is a result of disease diagnosis of the second subject P2.

As described above, the second learning result L2 is a learning result based on the second machine learning, and thus the disease diagnosis result determination unit 6 makes a determination based on the second machine learning.

The first learning result supply unit 7 supplies the first learning result L1 to the disease diagnosis result determination device 1. The second learning result supply unit 8 supplies the second learning result L2 to the disease diagnosis result determination device 1. The health examination data supply unit 9 supplies the health examination data MC to the disease diagnosis result determination device 1. The first learning result supply unit 7, the second learning result supply unit 8, and the health examination data supply unit 9 may each be, for example, a human interface device such as a keyboard, a tablet, or a scanner, may be an information storage device such as a server, or may be a portable terminal device such as a smart phone.

In addition, a large number of cases, each including the following as one set: health examination data; the results of a brain examination such as an MRI; and a diagnosis by a medical specialist, are collected from medical institutions and used as the teacher health examination data and teacher brain examination results of the first subject P1, and the teacher brain examination results and teacher disease diagnosis results of the third subject P3.

The presentation unit 10 is, for example, a display or a printer, and presents, by means of presentation such as display or printing, the disease diagnosis result D output from the disease diagnosis result determination unit 6 included in the disease diagnosis result determination device 1.

Note that the presentation unit 10 may be a storage device such as a network server. In this case, the presentation unit 10 stores the disease diagnosis result D supplied from the disease diagnosis result determination unit 6, and supplies the stored disease diagnosis result D to other devices.

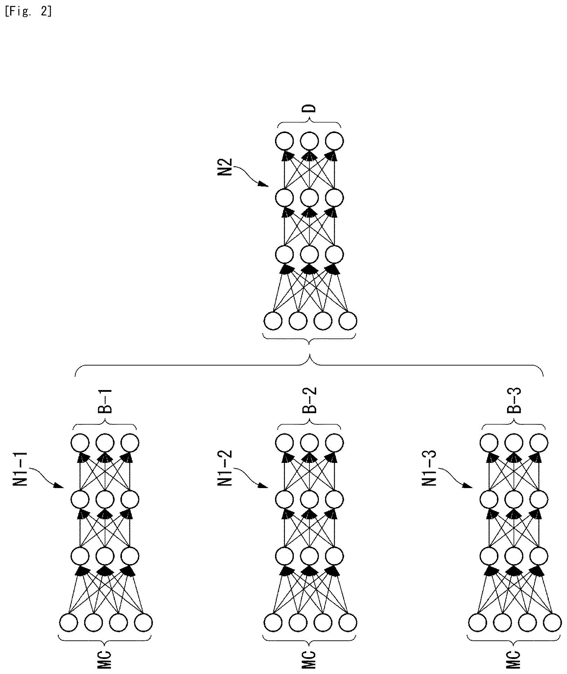

Fig. 2 is a diagram illustrating an example of the configuration of a neural network used for machine learning according to the present embodiment. A first neural network N1-1, a first neural network N1-2, and a first neural network N1-3 are each a deep learning neural network used for the first machine learning. The first machine learning is executed using the first neural network N1-1, the first neural network N1-2, and the first neural network N1-3 in parallel. A second neural network N2 is a deep learning neural network used for the second machine learning. In the disease diagnosis result determination device 1, machine learning is executed in two stages of the first machine learning and the second machine learning. The second machine learning, when executed, uses an output result of the first machine learning as an input. Put simply, the first machine learning and the second machine learning are executed in series.

Deep learning is a technique of machine learning using a multilayer neural network (a neural network with two or more hidden layers). The first neural network N1-1, the first neural network N1-2, the first neural network N1-3, and the second neural network N2 are each a neural network called a feedforward network. A feedforward network is a neural network in which information propagates in one direction from the input layer to the output layer.

In each of the first neural network N1-1, the first neural network N1-2, and the first neural network N1-3, when the health examination data MC is input to the input layer, the brain examination result determination result B is output from the output layer. In the present embodiment, the first neural network N1-1, the first neural network N1-2, and the first neural network N1-3 are the same in the configuration, except for the number of units provided in the output layer. Each of the first neural network N1-1, the first neural network N1-2, and the first neural network N1-3 has two hidden layers and 400 units for each hidden layer by way of example. Note that the number of hidden layers and the number of units in each hidden layer may differ among the first neural network N1-1, the first neural network N1-2, and the first neural network N1-3.

The number of units provided in the input layer of each of the first neural network N1-1, the first neural network N1-2, and the first neural network N1-3 depends on the number of types of data included in the health examination data MC and the expression of those data. Note that the data included in the health examination data MC is expressed as a numerical value or a value of a classified class according to the type.

The parameters (weights and biases) of the first neural network N1-1 are determined by learning performed based on the brain examination learning results LB-1. In the first neural network N1-1, when the health examination data MC is input to the input layer, an MMSE score is output from the output layer. The output layer of the first neural network N1-2 has the number of units corresponding to the expression of the MMSE score. The MMSE score is expressed by a number from 0 to 30, for example. Accordingly, the output layer of the first neural network N1-1 has 31 units.

The first neural network N1-2 is determined by learning performed based on the brain examination learning results LB-2. In the first neural network N1-2, when the health examination data MC is input to the input layer, a GM-BHQ is output from the output layer. The output layer of the first neural network N1-2 has the number of units corresponding to the expression of the GM-BHQ.

The first neural network N1-3 is determined by learning performed based on the brain examination learning results LB-3. In the first neural network N1-3, when the health examination data MC is input to the input layer, an FA-BHQ is output from the output layer. The output layer of the first neural network N1-3 has the number of units corresponding to the expression of the FA-BHQ.

Note that each of the GM-BHQ and FA-BHQ has a value related to a subject's age. The value of each of the GM-BHQ and FA-BHQ tends to decrease with age, with an average value of 100 for all ages. If the value of each of the GM-BHQ and FA-BHQ is lower than the value for the corresponding age, it indicates that the degree of brain health is low. The value of each of the GM-BHQ and FA-BHQ output from the output layer is expressed as a value for the age of the second subject P2.

The parameters (weights and biases) of the second neural network N2 are determined by learning performed based on the second learning result L2. In the second neural network N2, when the brain examination result determination result B is input to the input layer, a disease diagnosis result D is output from the output layer.

To the input layer of the second neural network N2, the outputs from the output layers of the first neural network N1-1, the first neural network N1-2, and the first neural network N1-3 are directly input. Accordingly, the number of units included in the input layer of the second neural network N2 is equal to the sum of the number of units included in the input layers of the first neural network N1-1, the first neural network N1-2, and the first neural network N1-3.

The output layer of the second neural network N2 has the number of units corresponding to the expression of the disease diagnosis result D. As described above, in the present embodiment, the disease diagnosis result D is classified into three classes and expressed, so that the output layer of the second neural network N2 has three units.

The second neural network N2 has two hidden layers and 400 units for each hidden layer by way of example. The configuration of the hidden layers of the second neural network N2 may be the same as or different from the configuration of the hidden layers of each of the first neural network N1-1, the first neural network N1-2, and the first neural network N1-3.

In the present embodiment, the types and values in the teacher health examination data are common among the brain examination learning results LB-i. In other words, in the present embodiment, a brain examination learning result LB-i is generated using common teacher health examination data among the brain examination learning results LB-i.

Note that a brain examination learning result LB-i may be generated using different teacher health examination data among the brain examination learning results LB-i. For example, each brain examination learning result LB-i may be generated by executing the first machine learning using different values of teacher health examination data among the brain examination learning results LB-i. Further, the types or the number of types in the teacher health examination data may differ among the brain examination learning results LB-i. Further, the number of pairs of teacher health examination data and teacher brain examination result may differ among the brain examination learning results LB-i.

For example, for the brain examination learning result LB-1, which corresponds to an MMSE score, age and a result of basic blood examination may be used as teacher health examination data, and 200 pieces of data may be used. For the brain examination learning result LB-2, which corresponds to a GM-BHQ, height, dosage history, and electrocardiogram may be used as teacher health examination data, and 1200 pieces of data may be used. For the brain examination learning result LB-3, which corresponds to an FA-BHQ, body weight, waist circumference, smoking habit, and taste examination may be used as teacher health examination data, and 500 pieces of data may be used.

In the case where the type or the number of types in the teacher health examination data differs among the brain examination learning results LB-i, the number of units included in the input layer of each of the first neural network N1-1, the first neural network N1-2, and the first neural network N1-3 differs depending on the type or the number of types in the teacher health examination data.

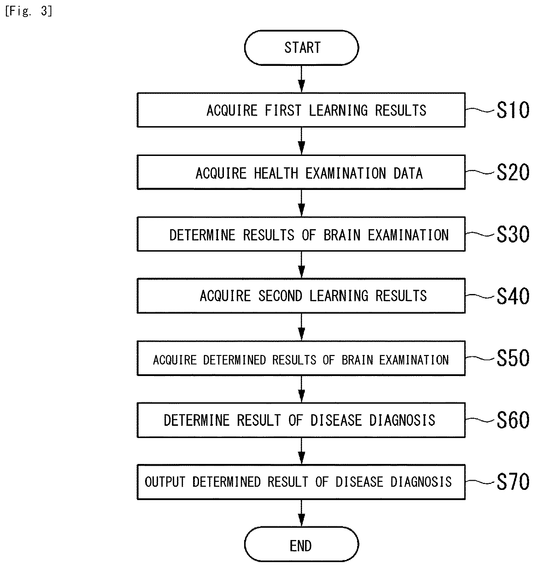

Fig. 3 illustrates an example of a determination step of the disease diagnosis result determination device 1 according to the present embodiment.

Step S10: The first learning result acquisition unit 2 acquires the first learning result L1 supplied from the first learning result supply unit 7. The first learning result acquisition unit 2 supplies the acquired first learning result L1 to the brain examination result determination unit 4.

Step S20: The health examination data acquisition unit 3 acquires the health examination data MC supplied from the health examination data supply unit 9. The health examination data acquisition unit 3 supplies the acquired health examination data MC to the brain examination result determination unit 4.

Step S30: The brain examination result determination unit 4 determines the brain examination result determination result B based on the first learning result L1 acquired by the first learning result acquisition unit 2 and the health examination data MC acquired by the health examination data acquisition unit 3. The brain examination result determination unit 4 supplies the determined brain examination result determination result B to the disease diagnosis result determination unit 6.

Step S40: The second learning result acquisition unit 5 acquires the second learning result L2 supplied from the second learning result supply unit 8. The second learning result acquisition unit 5 supplies the acquired second learning result L2 to the disease diagnosis result determination unit 6.

Step S50: The second learning result acquisition unit 5 acquires the brain examination result determination result B determined by the brain examination result determination unit 4.

Step S60: The disease diagnosis result determination unit 6 determines the disease diagnosis result D based on the second learning result L2 acquired by the second learning result acquisition unit 5 and the brain examination result determination result B determined by the brain examination result determination unit 4.

Step S70: The disease diagnosis result determination unit 6 outputs the determined disease diagnosis result D to the presentation unit 10.

Thus, the disease diagnosis result determination device 1 ends the determination step.

Note that when the brain examination result determination unit 4 cannot acquire part of the health examination data MC, the brain examination result determination unit 4 may use statistics of health examination data from a plurality of fourth subjects P4 to make the determination. Specifically, when part of the health examination data MC cannot be acquired, the brain examination result determination unit 4 may determine the brain examination result determination result B based on the first learning result L1 and the health examination data MC by using statistics of health examination data from a plurality of fourth subjects P4 as part of the health examination data MC. The statistic of health examination data is, for example, an average value, a median value, or the like. The fourth subjects P4 are subjects for which health examination data for interpolating the health examination data MC is provided when part of the health examination data MC is missing.

Note that in the present embodiment, an example in which the disease is cognitive impairment has been described by way of example, but the embodiment is not limited to this. A result of brain disease diagnosis such as depression as a disease may be determined in addition to or instead of cognitive impairment. Further, the disease diagnosis result determination device 1 may determine a result of disease diagnosis other than a brain disease.

As described above, the disease diagnosis result determination device 1 according to the present embodiment includes the brain examination result determination unit 4 and the disease diagnosis result determination unit 6.

The brain examination result determination unit 4 determines, based on the first learning result L1 for which a relationship between health examination data from the first subject P1 and a result of brain examination of the first subject P1 is learned in advance and based on the health examination data MC on the second subject P2, a result of brain examination of the second subject P2 (the brain examination result determination result B in the present embodiment).

The disease diagnosis result determination unit 6 determines, based on the second learning result L2 for which a relationship between a result of brain examination of the third subject P3 and a result of disease diagnosis of the third subject P3 is learned in advance and based on the result of brain examination of the second subject P2 (the brain examination result determination result B in the present embodiment) determined by the brain examination result determination unit 4, a result of disease diagnosis of the second subject P2 (the disease diagnosis result D in the present embodiment).

With this configuration, the disease diagnosis result determination device 1 according to the present embodiment can determine a result of brain examination from the health examination data MC, and further determine a result of disease diagnosis from the determined result of brain examination, so that it is possible to improve the accuracy of determination of the result of disease diagnosis.

Further, in the disease diagnosis result determination device 1, the items included in the health examination data MC may be any as long as they are examination items for various types of health examinations (physical checkups) such as specific health examinations. Therefore, it is possible to improve the accuracy of determination of the result of disease diagnosis, compared to the case where the determination is made based only on limited items such as age and blood data.

In the disease diagnosis result determination device 1, the results of brain examination determined from the health examination data MC may be for any type of brain examination. Therefore, it is possible to use brain anatomical and objective indices such as indices for MRI images and thus improve the accuracy of determination of the result of disease diagnosis, compared to the case of using an index based on a subjective evaluation method such as a questionnaire test (MMSE, etc.) score for a result of brain examination.

After determining a result of brain examination from the health examination data MC, the disease diagnosis result determination device 1 can determine a result of disease diagnosis from the result of brain examination so as to allow a doctor to make an integrated diagnosis of dementia from the result of brain examination. Therefore, the determination process using machine learning (deep learning) becomes similar to the diagnosis made by a medical specialist, and the process in which the result of disease diagnosis is determined using machine learning (deep learning) can be visualized.

In addition, the disease diagnosis result determination device 1 uses health examination data, so that systemic metabolic disorders that are at risk of dementia can be identified for each individual second subject P2. Therefore, this has an advantage of being able to provide a custom-made lifestyle improvement program (meal exercise therapy, etc.). Therefore, the disease diagnosis result determination device 1 enables efficient prevention of dementia, and the use of the disease diagnosis result determination device 1 results in an expected increase in incentives for life improvement.

The disease diagnosis result determination device 1 is intended to utilize regularly performed health examinations and to determine with high accuracy the risk of developing a disease such as dementia by using machine learning such as deep learning. In a conventional method, deep learning is used to predict cognitive impairment in a manner that age and blood data are used for the input layer and a questionnaire test (MMSE) score is output from the output layer. In such a conventional method, the items for the input layer and the output layer are limited, and the questionnaire test whose score is output from the output layer is a subjective examination method, so there is a problem in the prediction accuracy of cognitive impairment. Furthermore, the prediction process from the input layer to the output layer of deep learning is a black box, which is an obstacle to the application of deep learning to medical care.

Further, the result of brain examination used in the disease diagnosis result determination device 1 according to the present embodiment includes the results of a plurality of types of brain examinations. The first learning result L1 includes a plurality of types of learning results for the respective types of brain examinations (the brain examination learning result LB-1, the brain examination learning result LB-2, and the brain examination learning result LB-3 in the present embodiment). The brain examination result determination unit 4 determines each of results of a plurality of types of brain examinations of the second subject P2 based on the plurality of types of learning results for the respective types of brain examinations (the brain examination learning result LB-1, the brain examination learning result LB-2, and the brain examination learning result LB-3 in the present embodiment) and based on the health examination data MC of the second subject P2.

With this configuration, the disease diagnosis result determination device 1 according to the present embodiment can use the results of the plurality of types of brain examinations to make the determination, so that it is possible to improve the accuracy of determination of the result of disease diagnosis, compared to the case of using the result of one type of brain examination.

Further, in the disease diagnosis result determination device 1 according to the present embodiment, the brain examination result determination unit 4 determines a result of brain examination of the second subject P2 (the brain examination result determination result B in the present embodiment) based on the first learning result L1 and the health examination data MC on the second subject P2 by using statistics of health examination data from the plurality of fourth subjects P4 as part of the health examination data MC on the second subject P2.

With this configuration, even when part of the health examination data MC of the second subject P2 cannot be acquired, the disease diagnosis result determination device 1 according to the present embodiment can use the statistic of health examination data from the plurality of fourth subjects P4 in place of the part of the health examination data MC. Therefore, the disease diagnosis result determination device 1 can determine the result of disease diagnosis even when part of the health examination data MC on the second subject P2 cannot be acquired.

Modified examples of the present embodiment will be described below.

### Modified Example 1

In Modified Example 1, a case will be described in which when the results for which one or some of the plurality of types of brain examinations of the second subject P2 are performed in advance can be acquired, for those results of brain examinations, the results for which actual brain examinations are performed in advance are used to determine a result of disease diagnosis instead of the results of brain examinations obtained using the first machine learning.

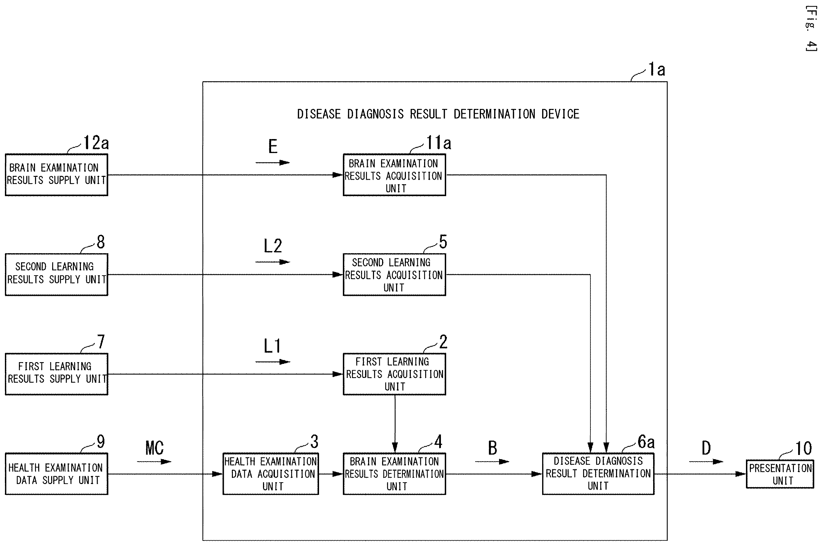

Fig. 4 is a diagram illustrating an example of the configuration of a disease diagnosis result determination device 1a according to Modified Example 1. The disease diagnosis result determination device 1a (Fig. 4) according to the present modified example is different from the disease diagnosis result determination device 1 (Fig. 1) according to the above-described embodiment in a disease diagnosis result determination unit 6a, a brain examination result acquisition unit 11a, and a brain examination result supply unit 12a. The same reference numerals are assigned to the same components as those of the above-described embodiment, and the descriptions of the same components and operations will be omitted.

The disease diagnosis result determination device 1a includes the first learning result acquisition unit 2, the health examination data acquisition unit 3, the brain examination result determination unit 4, the second learning result acquisition unit 5, the disease diagnosis result determination unit 6a, and the brain examination result acquisition unit 11a.

The brain examination result acquisition unit 11a acquires a brain examination result E supplied from the brain examination result supply unit 12a. The brain examination result E is a result for which one or some of the plurality of types of brain examinations of the second subject P2 are performed in advance.

For one or some of the plurality of types of brain examinations, the disease diagnosis result determination unit 6a uses the brain examination result E acquired by the brain examination result acquisition unit 11a to make the determination in place of the brain examination result determination result B determined by the brain examination result determination unit 4.

The brain examination result supply unit 12a supplies the brain examination result E to the disease diagnosis result determination device 1a. The brain examination result supply unit 12a may be, for example, a human interface device such as a keyboard, a tablet, or a scanner, or may be an information storage device such as a server.

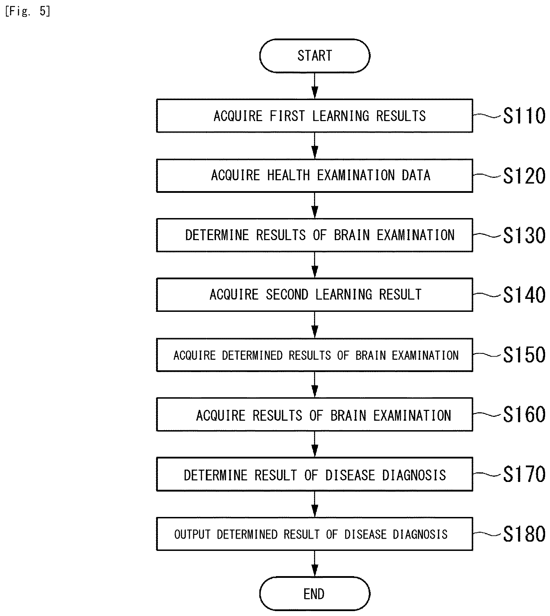

Fig. 5 illustrates an example of a determination step of the disease diagnosis result determination device 1a according to the present embodiment. Note that the processes from step S110 to step S140, step S160, and step S180 are the same as the processes from step S10 to step S40, step S50, and step S70 in Fig. 3, and thus the description thereof will be omitted.

Step S150: The brain examination result acquisition unit 11a acquires the brain examination result E supplied from the brain examination result supply unit 12a. The brain examination result acquisition unit 11a supplies the acquired brain examination result E to the disease diagnosis result determination unit 6a. The brain examination result E includes, for example, an FA-BHQ of the plurality of types of brain examinations.

Step S170: The disease diagnosis result determination unit 6 determines the disease diagnosis result D based on the second learning result L2 acquired by the second learning result acquisition unit 5, the brain examination result determination result B determined by the brain examination result determination unit 4, and the brain examination result E acquired by the brain examination result acquisition unit 11a. For one or some of the plurality of types of brain examinations, the disease diagnosis result determination unit 6a uses the brain examination result E acquired by the brain examination result acquisition unit 11a to make the determination in place of the brain examination result determination result B determined by the brain examination result determination unit 4.

The disease diagnosis result determination unit 6 uses, for example, an MMSE score, a GM-BHQ, and an FA-BHQ acquired by the brain examination result acquisition unit 11a in the brain examination result determination result B determined by the brain examination result determination unit 4 to determine the disease diagnosis result D. Note that the brain examination result determination unit 4 may do without making a determination of brain examination results in the brain examination result determination result B, which correspond to those in the brain examination result E acquired by the brain examination result acquisition unit 11a.

As described above, the disease diagnosis result determination device 1a according to the present modified example includes the brain examination result acquisition unit 11a. The brain examination result acquisition unit 11a acquires the brain examination result E for which one or some of the plurality of types of brain examinations of the second subject P2 are performed in advance. For one or some of the plurality of types of brain examinations, the disease diagnosis result determination unit 6 uses the brain examination result E acquired by the brain examination result acquisition unit 11a to make the determination in place of the result of brain examination of the second subject P2 (the brain examination result determination result B in the present embodiment) determined by the brain examination result determination unit 4.

With this configuration, when a result of brain examination for which brain examination(s) are performed in advance can be acquired, the disease diagnosis result determination device 1a according to the present modified example can use that result of brain examination to make the determination, so that it is possible to improve the accuracy of determination of the result of disease diagnosis by using the result of actual brain examination. It can do.

As described above, the disease diagnosis result determination device 1a can make the determination using the health examination data MC without actually performing a brain examination such as taking an MRI image. On the other hand, for example, when the brain examination result determination result B indicates that the result of brain examination is not good in the first machine learning, the corresponding brain examination may be performed only on high-risk subjects, and the brain examination is performed, so that their results of brain examination thus obtained are used for determination to improve the accuracy of the determination.

### Modified Example 2

In some cases, it is desired to confirm the process through which the result of disease diagnosis is determined by the disease diagnosis result determination device. In Modified Example 2, a case will be described in which when it is desired to confirm the brain examination result determination result used to determine a result of disease diagnosis, that brain examination result determination result is presented.

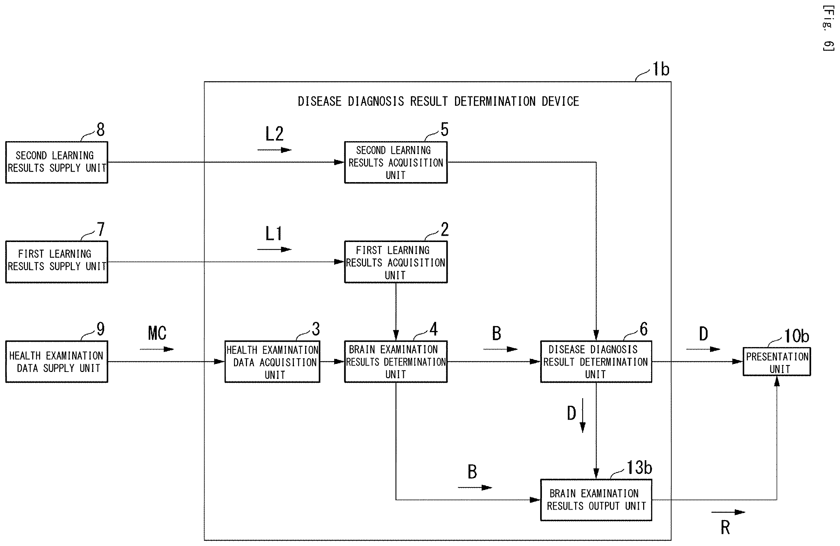

Fig. 6 is a diagram illustrating an example of the configuration of a disease diagnosis result determination device 1b according to Modified Example 2. The disease diagnosis result determination device 1b (Fig. 6) according to the present modified example is different from the disease diagnosis result determination device 1 (Fig. 1) according to the above-described embodiment in a brain examination result output unit 13b and a presentation unit 10b. The same reference numerals are assigned to the same components as those of the above-described embodiment, and the descriptions of the same components and operations will be omitted.

The disease diagnosis result determination device 1b includes the first learning result acquisition unit 2, the health examination data acquisition unit 3, the brain examination result determination unit 4, the second learning result acquisition unit 5, the disease diagnosis result determination unit 6, and the brain examination result output unit 13b.

The brain examination result output unit 13b outputs determination process information R after the disease diagnosis result determination unit 6 determines the disease diagnosis result D. The determination process information R is a set of the result of brain examination of the second subject P2 used to determine the disease diagnosis result D and the disease diagnosis result D.

The presentation unit 10b presents the determination process information R output from the brain examination result output unit 13b by means of presentation such as display and printing.

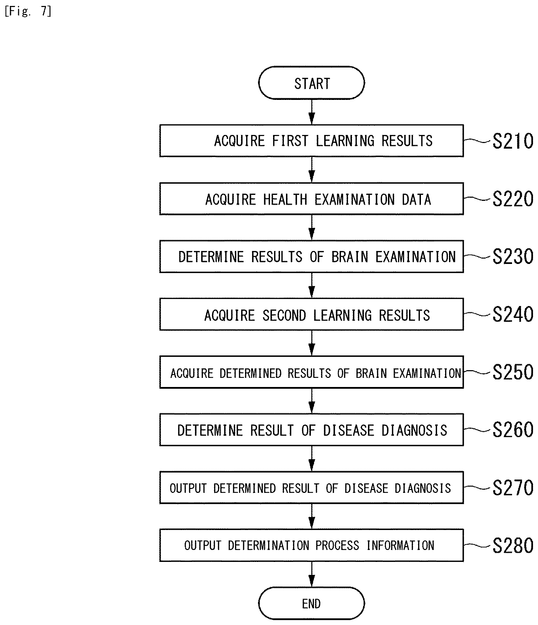

Fig. 7 illustrates an example of a determination step of the disease diagnosis result determination device 1b according to the present embodiment. Note that the processes from step S210 to step S270 are the same as the processes from step S10 to step S70 in Fig. 3, and thus the description thereof will be omitted.

Step S280: The brain examination result output unit 13b outputs the determination process information R to the presentation unit 10b. Specifically, the brain examination result output unit 13b outputs the determination process information R after the disease diagnosis result determination unit 6 determines the disease diagnosis result D in step 270.

As described above, the disease diagnosis result determination device 1b according to the present modified example includes the brain examination result output unit 13b. After the disease diagnosis result determination unit 6 determines the result of disease diagnosis (the disease diagnosis result D in the present embodiment), the brain examination result output unit 13b outputs a set of the result of brain examination of the second subject P2 (the brain examination result determination result B in the present embodiment) used to determine that result of diagnosis (the disease diagnosis result D in the present embodiment) and that result of diagnosis (the disease diagnosis result D in the present embodiment).

With this configuration, in the disease diagnosis result determination device 1b according to the present modified example, it is possible to confirm the result of brain examination used to determine the result of disease diagnosis, so that the determination process can be confirmed. As described above, hitherto, the prediction process from the input layer to the output layer of deep learning is a black box, which is an obstacle to the application of deep learning to medical care. By contrast, the disease diagnosis result determination device 1b can visualize the process of estimating the output layer from the input layer of the deep learning method.

Note that, in the embodiment described above, an example has been described in which the result of brain examination includes results of a plurality of types of brain examinations, but the embodiment is not limited to this. The result of brain examination may include of only one type of brain examination (for example, only an MMSE result).

Note that, in the embodiment described above, an example has been described in which each of the first machine learning and the second machine learning are deep learning, but the present embodiment is not limited to this. Any other machine learning may be used as each of the first machine learning and the second machine learning as long as it is supervised learning. The first machine learning and the second machine learning to be used may be different types of machine learning.

Note that, in the embodiment described above, an example has been described in which the disease diagnosis result determination device 1 acquires the first learning result L1 and the second learning result L2 from external devices (the first learning result supply unit 7 and the second learning result supply unit 8), but the embodiment is not limited to this. The disease diagnosis result determination device 1 may execute either or both of the first machine learning and the second machine learning. The disease diagnosis result determination device 1 may include either or both of a first learning unit and a second learning unit. The first learning unit executes the first machine learning and supplies the first learning result L1 to the first learning result acquisition unit 2. The second learning unit executes the second machine learning and supplies the second learning result L2 to the second learning result acquisition unit 5.

Note that, in the embodiment described above, examples have been described in which the disease diagnosis result determination devices 1, 1a, and 1b each include the brain examination result determination unit 4 and the disease diagnosis result determination unit 6, and the disease diagnosis result determination devices 1, 1a, and 1b each execute the first machine learning and the second machine learning, but the embodiment is not limited to these. The first machine learning and the second machine learning may be executed by separate determination devices. In that case, for example, the determination device that executes the first machine learning includes the first learning result acquisition unit 2, the health examination data acquisition unit 3, and the brain examination result determination unit 4. Further, in that case, the determination device that executes the second machine learning includes the second learning result acquisition unit 5 and the disease diagnosis result determination unit 6, and acquires the brain examination result determination result from the determination device that executes the first machine learning to determine a result of disease diagnosis.

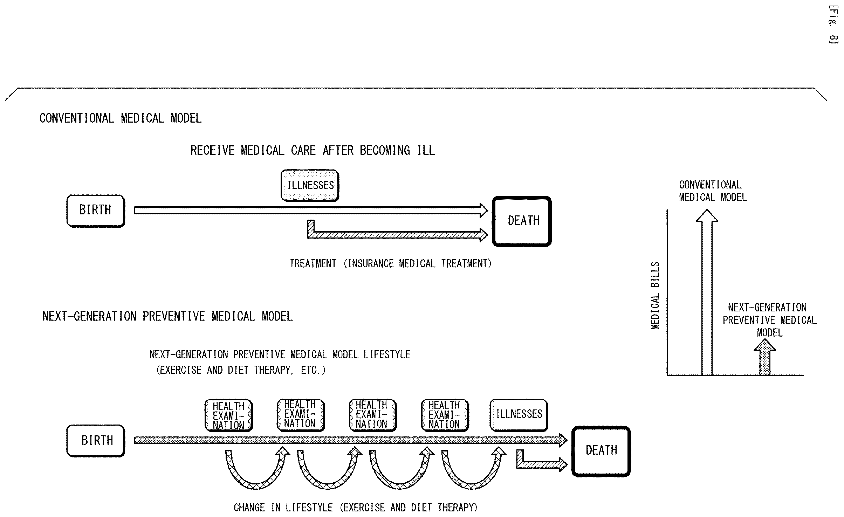

Referring to Fig. 8, an example of a next-generation preventive medical model according to an embodiment of the present invention is illustrated. The conventional medical models assume that a person becomes ill and then visits a medical institution to receive a medical treatment (insurance medical treatment). By contrast, the use of the disease diagnosis result determination devices 1, 1a, and 1b according to the embodiments described above makes it possible to determine a result of disease diagnosis based on the data from health examinations that a subject receives every year in a healthy state, which helps the subject to improve his/her lifestyle in a healthy state by referring to the result of disease diagnosis. This will prevent illnesses, extending healthy life expectancy and also reducing medical expenses.

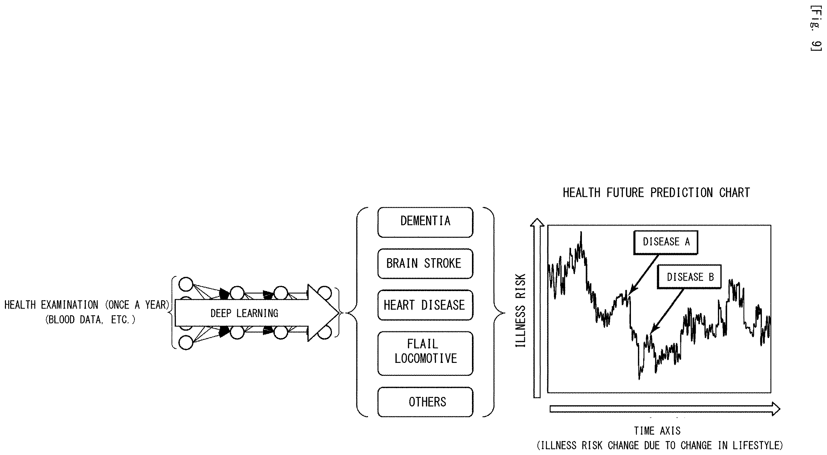

Fig. 9 illustrates an example of a health future prediction chart according to the present embodiment. The health future prediction chart is a plot of risks of illnesses based on the results of disease diagnosis obtained by the disease diagnosis result determination devices 1, 1a, and 1b according to the embodiments described above, with a time axis. The time indicated by this time axis is the time when the health examination data was acquired.

In the disease diagnosis result determination devices 1, 1a, and 1b according to the embodiments described above, various diseases can be predicted from health examination data by changing the type of disease diagnosis whose result is to be obtained corresponding to the output layer of the neural network used for deep learning. Furthermore, by continuously acquiring health examination data for a long period of time and learning the acquired health examination data, the illness risk of various diseases can be predicted. Since the risk of illness changes every year depending on lifestyle, the health future prediction chart enables visualization of lifestyle-change effects and increases incentives for the change in lifestyle.

### Example 1

Next, an example of Modified Example 2 described above will be described. In the present modified example, a feedforward deep learning neural network as illustrated in Fig. 2 was used. In the present modified example, blood examination data and age were used as the health examination data MC to be input to the input layer. In the present modified example, a GM-BHQ from MRI was used as the brain examination result determination result B output from the output layer of the first neural network. Learning was performed by the first neural network using brain dock data of 1799 cases. The accuracy of prediction was verified by cross-validation.

The above-mentioned brain dock data includes a set of age, blood examination data, and GM-BHQ. The brain dock data is data indicating the results of brain dock examinations of 1799 brain dock patients with an average age of 62.0 ± 13.1 years. The GM-BHQ, which is a BHQ of brain gray matter (GM), was used as a brain atrophy index indicating the degree of brain atrophy. The GM-BHQ was measured based on imaging results from a 3T-MR device.

The blood examination data used as the health examination data MC includes the following 26 items. Specifically, the blood examination data includes 26 items: total protein (TP), albumin, A/G ratio, total bilirubin, GOT, GPT, γ-GTP, ALP, WBC, RBC, Hb, Ht, PLT, HCV, total cholesterol, neutral fat, HDL cholesterol, LDL cholesterol, calculated LDL cholesterol, blood sugar, HbA1c, fibrinogen, urea nitrogen (BUN), creatine (Cr), uric acid, and amylase.

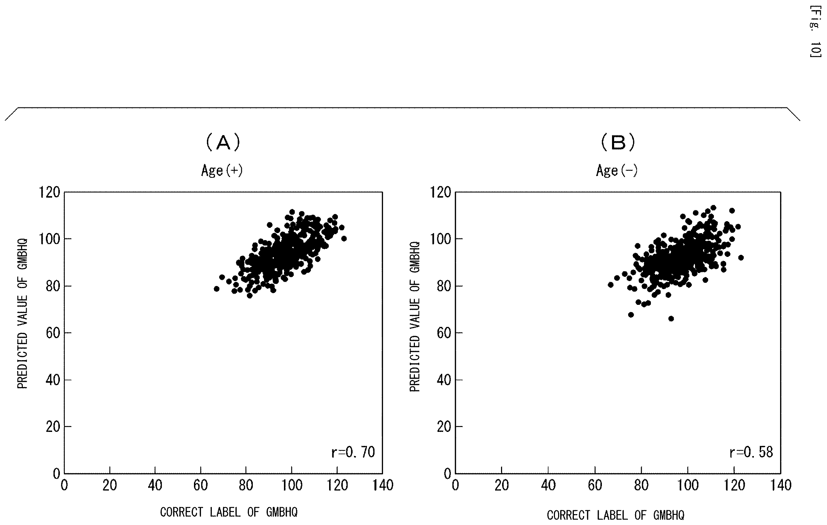

Fig. 10 illustrates an example of a correlation between actual measured values (correct labels) and predicted values of GM-BHQ according to the present example. Note that the actual measured values (correct labels) and the predicted values of GM-BHQ correspond to the determination process information R output by the brain examination result output unit 13b included in the disease diagnosis result determination device 1b (Fig. 6) described in Modified Example 2.

Fig. 10 (A) indicates a correlation when age is included in the health examination data MC to be input to the input layer. The actual measured values of GM-BHQ (correct label) are the values of GM-BHQ included in the brain dock data of 1799 cases described above. The predicted values of GM-BHQ are the values of GM-BHQ output in response to inputting the health examination data MC to the first neural network learned using the brain dock data.

The correlation coefficient for the correlation indicated in Fig. 10(A) was 0.70, and the p-value was less than 0.001, and therefore, a significant positive correlation was observed between the measured values and the predicted values. For comparison, Fig. 10(B) indicates a correlation when age is not included in the health examination data MC. The correlation coefficient for the correlation indicated in Fig. 10(B) was 0.58, and the p-value was less than 0.001, and therefore, a significant positive correlation was observed between the measured values and the predicted values even when age is not included in the health examination data MC.

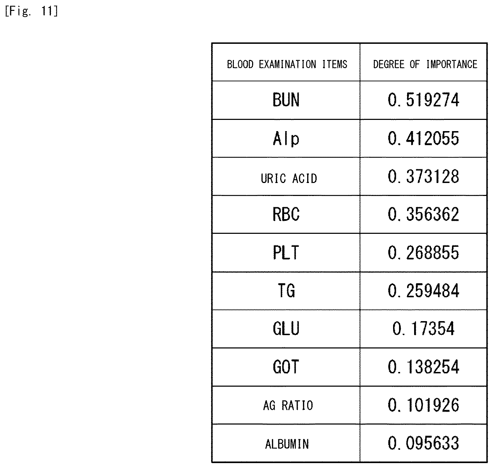

In addition, the degrees of importance to the prediction results were calculated for 26 items included in the blood examination data used in the health examination data MC. Fig. 11 indicates the degrees of importance for the top 10 items. The item of age was the most important to the prediction results, followed by the item of urea nitrogen.

The above results indicate that the use of deep learning (a deep neural network using a feedforward network) makes it possible to predict not only cognitive function changes but also structural changes in the brain (brain atrophy) from the basic blood examination data, and also indicate that the two-stage deep learning in the disease diagnosis result determination devices 1, 1a, and 1b according to the embodiments can be applied to the diagnosis of dementia. The above results also suggest that brain anatomy is closely related to systemic metabolic disorders. Predicting structural changes in the brain and cognitive function based on the basic blood examination data by the two-stage deep learning may be applied as a screening test for dementia.

Note that part of the disease diagnosis result determination device 1, 1a, or 1b according to the embodiments described above, for example, the brain examination result determination unit 4, the disease diagnosis result determination unit 6, the brain examination result acquisition unit 11a, and the brain examination result output unit 13b may be implemented by a computer. In that case, a program for implementing their control functions may be recorded in a computer-readable recording medium, and the program recorded in this recording medium may be read into a computer system and executed. The term "computer system" as used herein is a computer system built in the disease diagnosis result determination device 1, 1a, or 1b, and includes an OS and hardware such as peripheral devices. The term "computer-readable recording medium" refers to a portable medium such as a flexible disc, a magneto-optical disc, a ROM, and a CD-ROM, or a storage device such as a hard disc built into the computer system. Furthermore, the term "computer-readable recording medium" may refer to a medium that dynamically stores a program for a short period of time, such as a communication line for transmitting a program via a network such as the Internet or a communication line such as a telephone line, or a medium that stores a program for a certain period of time, such as a volatile memory inside the computer system that serves as a server or client in the above case of transmitting the program. Further, the program may be for implementing part of the functions described above, or may be capable of implementing the functions described above in combination with a program already recorded in the computer system.

Further, part or all of the disease diagnosis result determination device 1, 1a, or 1b according to the embodiments described above may be implemented as an integrated circuit such as large scale integration (LSI). Each functional block in the disease diagnosis result determination device 1, 1a, or 1b may be an individual processor, or a part or all of the functional blocks may be an integrated processor. Further, the technique of circuit integration is not limited to LSI, but may be implemented by a dedicated circuit or a general-purpose processor. Further, when an integration circuit technology that replaces LSI appears due to advances in semiconductor technology, an integrated circuit based on that technology may be used.

Although the embodiments of the present invention have been described in detail above with reference to the drawings, the specific configurations are not limited to the above, and various design changes and the like can be made without departing from the scope and spirit of the present invention.

### INDUSTRIAL APPLICABILITY

In the present invention, the first machine learning and the second machine learning are used, but each machine learning (deep learning) algorithm uses an existing algorithm (such as a feedforward neural network), and therefore the development of the algorithm itself is not necessary. Further, cooperation with the Japan Brain Dock Society makes it not difficult to collect a large number of cases each including a set of health examination data, a result of brain examination such as MRI, and diagnosis by a medical specialist.

The use of the present invention makes it possible to provide inexpensive large-scale screening tests for diseases such as dementia using health examination data. Since the present invention uses only health examination data, there is no need to collect blood, and there is an advantage that the risk of diseases such as dementia can be determined in daily life such as usage at non-medical facility such as sports gym and use of a smartphone.

Making use of such an advantage and combining the present invention with dementia insurance that has recently been put on the market makes it possible to detect dementia at an early stage. Furthermore, combination with specific health examinations and specific health guidance (for those aged 40 to 75 who have insurance coverage and dependents) makes it possible to suppress or delay the onset of dementia at municipal and national levels. This is an expectation of innovation in dementia measures, which have become a social problem.

### REFERENCE SIGNS LIST

- 1, 1a, 1b: Disease diagnosis result determination device
- 4: Brain examination result determination unit
- 6: Disease diagnosis result determination unit
- L1: First learning result
- L2: Second learning result
- MC: Health examination data
- B: Brain examination result determination result
- D: Disease diagnosis result

## Claims

1. A disease diagnosis result determination device, comprising:
a brain examination result determination unit configured to determine, based on a first learning result for which a relationship between health examination data from a first subject and a result of brain examination of the first subject is learned in advance and based on health examination data from a second subject, a result of brain examination of the second subject; and
a disease diagnosis result determination unit configured to determine, based on a second learning result for which a relationship between a result of brain examination of a third subject and a result of disease diagnosis of the third subject is learned in advance and based on the result of brain examination of the second subject determined by the brain examination result determination unit, a result of disease diagnosis of the second subject.

2. The disease diagnosis result determination device according to claim 1, wherein
the result of brain examination includes results of a plurality of types of brain examinations,
the first learning result includes a plurality of types of learning results for the respective types of brain examinations, and
the brain examination result determination unit is configured to determine, based on the plurality of types of learning results for the respective types of brain examinations and based on the health examination data from the second subject, each of the results of the plurality of types of brain examinations of the second subject.

3. The disease diagnosis result determination device according to claim 2, further comprising
a brain examination result acquisition unit configured to acquire a result of brain examination obtained by performing one or some of the types of brain examinations of the second subject in advance,
wherein the disease diagnosis result determination unit is configured to use the result of brain examination acquired by the brain examination result acquisition unit and make the determination for the one or some of the types of brain examinations, instead of the result of brain examination of the second subject determined by the brain examination result determination unit.

4. The disease diagnosis result determination device according to any one of claims 1 to 3, wherein
the brain examination result determination unit is configured to determine a result of brain examination of the second subject based on the first learning result and the health examination data from the second subject by using statistics of health examination data from a plurality of fourth subjects as part of the health examination data from the second subject.

5. The disease diagnosis result determination device according to any one of claims 1 to 4, further comprising
a brain examination result output unit configured to output, after the disease diagnosis result determination unit determines the result of disease diagnosis, a set of the results of brain examination of the second subject used to determine the result of disease diagnosis and the result of disease diagnosis.

6. A disease diagnosis result determination method, comprising:
a brain examination result determination step of determining, based on a first learning result for which a relationship between health examination data from a first subject and a result of brain examination of the first subject is learned in advance and based on health examination data from a second subject, a result of brain examination of the second subject; and
a disease diagnosis result determination step of determining, based on a second learning result for which a relationship between a result of brain examination of a third subject and a result of disease diagnosis of the third subject is learned in advance and based on the result of brain examination of the second subject determined by the brain examination result determination step, a result of disease diagnosis of the second subject.

7. A program for causing a computer to execute:
a brain examination result determination step of determining, based on a first learning result for which a relationship between health examination data from a first subject and a result of brain examination of the first subject is learned in advance and based on health examination data from a second subject, a result of brain examination of the second subject; and
a disease diagnosis result determination step of determining, based on a second learning result for which a relationship between a result of brain examination of a third subject and a result of disease diagnosis of the third subject is learned in advance and based on the result of brain examination of the second subject determined by the brain examination result determination step, a result of disease diagnosis of the second subject.
